# EUROPÄISCHE PATENTANMELDUNG

(11) **EP 4 841 303 A1**
(43) Veröffentlichungstag der Anmeldung: **18.11.2026**
(21) Anmeldenummer: 26175493.1
(22) Anmeldetag: 29.04.2026
(51) Int. Cl.: G01S 13/88, A61B 5/11, A61B 5/024, A61B 5/113, A61B 5/0507, G01S 7/41, G01S 13/86, G01S 17/86

(54) **RADARGERÄT MIT OPTISCHER POSITIONIERUNGSHILFE**

(30) Priorität: 16.05.2025 DE 102025119092
(71) Anmelder: Fraunhofer-Gesellschaft zur Förderung der angewandten Forschung e.V., 80686 München (DE)
(72) Erfinder: VOX, Jan, 26129 Oldenburg (DE); APPELL, Jens-Ekkehart, 26129 Oldenburg (DE)
(74) Vertreter: Fischer, Florian

(57) **Zusammenfassung**

Die Erfindung betrifft eine Vorrichtung (100) zur kontaktlosen, radarbasierten Erfassung von Distanzen zu einer zu untersuchenden Objektoberfläche (110). Die Vorrichtung (100) weist ein von der Objektoberfläche (110) beabstandetes Radargerät (120) auf. Das Radargerät (120) beinhaltet einen Radartransmitter (121) zum Aussenden von Primär-Radarwellen (131) auf die Objektoberfläche (110) sowie einen Radarempfänger (122) zum Empfangen von an der Objektoberfläche (110) reflektierten Sekundär-Radarwellen (132). Eine Auswerteeinheit (130) führt, basierend auf den empfangenen Sekundär-Radarwellen (132), eine Distanzbestimmung zu der Objektoberfläche (110) aus. Das Radargerät (120) weist einen vordefinierten Radarmessbereich (140) auf, wobei eine optische Positionierungshilfe (150) ausgestaltet ist, um einem Anwender den ansonsten unsichtbaren Radarmessbereich (140) mit Hilfe von optischen Mitteln (210) zu visualisieren

## Beschreibung

Das technische Gebiet des hierin offenbarten Erfindungsgegenstands betrifft die kontaktlose radargestützte Ermittlung von Distanzen bzw. Abständen zu einer zu untersuchenden Objektoberfläche. Das hierin beschriebene innovative Konzept beschreibt dabei eine optische Positionierungshilfe, die es dem Anwender erlaubt, das Radargerät optimal zu der zu untersuchenden Objektoberfläche auszurichten, indem sie den ansonsten unsichtbaren Radarmessbereich des Radargeräts visualisiert.

Die radargestützte Distanzmessung zu einer zu untersuchenden Objektoberfläche kann mittels geeigneter Radargeräte erfolgen, die Primär-Radarwellen aussenden und die vom Objekt reflektierten Sekundär-Radarwellen empfangen. Anhand der Sekundär-Radarwellen kann die Entfernung des Objekts hochauflösend detektiert werden. Mit Hilfe von über einen bestimmten Zeitraum hinweg durchgeführten Distanzmessungen wird es beispielsweise möglich, zeitvariante Distanzunterschiede zu erfassen und hierüber auf Bewegungen der zu untersuchenden Objektoberfläche zu schließen.

Die radargestützte Erkennung von Bewegungen einer zu untersuchenden Objektoberfläche kann sowohl im industriellen Umfeld, wie z.B. bei Maschinen, Robotern und dergleichen, als auch im medizinischen bzw. klinischen Umfeld, z.B. zur Untersuchung von Bewegungen eines Körpers oder Körperteils, zur Erkennung oberflächlicher Hautbewegungen, usw. eingesetzt werden.

So hat beispielsweise die Anmelderin ein Gerät zur kontaktlosen radargestützten Messung von Vitalparametern bei einem menschlichen Körper entwickelt. Dabei kann anhand von mittels Radar detektieren Hautbewegungen auf Vitalparameter, wie z.B. den Herzschlag oder Puls rückgeschlossen werden. Auch eine Messung des Blutdrucks kann mittels Radarwellen kontaktlos erfolgen.

In aktuellen Publikationen zur radargestützten kontaktlosen Erfassung von Vitaldaten ist beschrieben, dass das Radargerät in fest vorgegebenen starren Positionen relativ zum Körper angeordnet wird. Dabei wird generell davon ausgegangen, dass sich der Körper stets innerhalb eines optimalen Messbereichs des Radargeräts befindet.

In [Hornig et al., 2024] wurden beispielsweise verschiedene Möglichkeiten zur Positionierung des Radargeräts relativ zum Körper einer schlafenden oder liegenden Person untersucht. Dabei wurde jedoch zur Vereinfachung angenommen, dass sich die zu untersuchende Person stets im optimalen Messbereich des Radargeräts befindet und das Radargerät somit flexibel um das Bett herum positioniert werden kann.

In realen Szenarien wurde jedoch festgestellt, dass diese vereinfachte Annahme nicht immer zutreffend ist, d.h. es ist eben gerade nicht immer möglich, das Radargerät frei um ein Bett herum oder in der jeweiligen Messumgebung zu positionieren. Zudem befindet sich eine zu untersuchende Person nicht immer genau an der angenommenen Stelle.

Da der Messbereich von Radargeräten, und insbesondere die Radarwellen selbst, für das menschliche Auge unsichtbar sind, ist der Ausbreitungspfad der Primär- und Sekundär-Radarwellen nicht erkennbar. Dadurch ist es für den Anwender schwierig, die optimale Positionierung, d.h. die Orientierung des Radargeräts relativ zu der zu untersuchenden Objektoberfläche, ausfindig zu machen. Dies kann dann wiederum gelegentlich dazu führen, dass das Radargerät nicht optimal zur Objektoberfläche ausgerichtet ist, sodass die radargestützten Messungen nicht mit ausreichender Genauigkeit, oder teilweise auch gar nicht, durchgeführt werden können.

Es ist daher Ziel des hierin beschriebenen innovativen Konzepts den Anwender dabei zu unterstützen, das Radargerät korrekt zu positionieren, damit es so zu der zu untersuchenden Objektoberfläche ausgerichtet ist, dass Messungen der vorgenannten Art möglich sind.

Dieses Ziel wird erreicht durch eine Vorrichtung gemäß Anspruch 1. Weitere Ausführungsformen und vorteilhafte Aspekte der erfindungsgemäßen Vorrichtung sind in den jeweils abhängigen Patentansprüchen genannt.

Gemäß dem hierin beschriebenen innovativen Konzept wird eine Vorrichtung zur kontaktlosen, radarbasierten Distanzmessung zu einer zu untersuchenden Objektoberfläche vorgeschlagen. Die Vorrichtung weist unter anderem ein von der Objektoberfläche beabstandetes Radargerät auf, wobei dieses Radargerät einen Radartransmitter zum Aussenden von Primär-Radarwellen auf die Objektoberfläche aufweist sowie einen Radarempfänger zum Empfangen von an der Objektoberfläche reflektierten Sekundär-Radarwellen. Die Vorrichtung weist außerdem eine Auswerteeinheit auf, die ausgestaltet ist, um basierend auf den empfangenen Sekundär-Radarwellen Abstände bzw. Distanzen zwischen dem Radargerät und der zu untersuchenden Objektoberfläche zu detektieren. Die Distanzmessung kann dann zum Beispiel in einigen Ausführungsformen dazu genutzt werden, um eine Bewegung der zu untersuchenden Objektoberfläche zu detektieren. Unterschiede bei den gemessenen Distanzen über die Zeit hinweg können beispielsweise darauf hindeuten, dass sich die Oberfläche relativ zum Radargerät bewegt bzw. bewegt hat. Das Radargerät weist einen vordefinierten Radarmessbereich auf, innerhalb dessen eine Distanzmessung zu der zu untersuchenden Objektoberfläche möglich ist. Außerhalb des Radarmessbereichs ist keine, oder aber nur eine ungenügend genaue, Distanzmessung möglich. Dabei ist eine erfolgreiche Distanzmessung abhängig von der Orientierung des Radargeräts relativ zu der zu untersuchenden Objektoberfläche. Um dem Benutzer die korrekte Ausrichtung des Radargeräts zu erleichtern, weist die Vorrichtung erfindungsgemäß eine optische Positionierungshilfe auf, die ausgestaltet ist, um einem Anwender den ansonsten unsichtbaren Radarmessbereich mit Hilfe von optischen Mitteln zu visualisieren.

Die visuelle Anzeige kann zum Beispiel auf der zu untersuchenden Objektoberfläche stattfinden, z.B. durch einen auf die Objektoberfläche gerichteten Lichtschein, der den vordefinierten Radarmessbereich des Radargeräts für den Anwender visualisiert. Dadurch wird der Radarmessbereich, der sich u.a. auf Basis der ausgesendeten Primär-Radarwellen und der empfangenen reflektierten Sekundär-Radarwellen bestimmt, für den Anwender sichtbar gemacht. Somit hat der Anwender einen visuellen Anhaltspunkt, der ihn befähigt, das Radargerät relativ zu der zu untersuchenden Objektoberfläche korrekt auszurichten, sodass die Distanzmessungen zu der zu untersuchenden Objektoberfläche mittels des Radargeräts erfolgreich durchgeführt werden können.

Einige Ausführungsbeispiele sind exemplarisch in der Zeichnung dargestellt und werden nachstehend erläutert. Es zeigen:
- Fig. 1: eine schematische seitliche Ansicht einer erfindungsgemäßen Vorrichtung gemäß einem Ausführungsbeispiel, und
- Fig. 2: eine schematische seitliche Ansicht einer erfindungsgemäßen Vorrichtung gemäß einem weiteren Ausführungsbeispiel.

Im Folgenden werden Ausführungsbeispiele mit Bezug auf die Figuren näher beschrieben, wobei Elemente mit derselben oder ähnlichen Funktion mit denselben Bezugszeichen versehen sind.

Verfahrensschritte, die im Rahmen der vorliegenden Offenbarung abgebildet bzw. beschrieben sind, können auch in einer anderen als der abgebildeten beziehungsweise beschriebenen Reihenfolge ausgeführt werden. Außerdem sind Verfahrensschritte, die ein bestimmtes Merkmal einer Vorrichtung betreffen mit ebendiesem Merkmal der Vorrichtung austauschbar, was ebenso anders herum gilt.

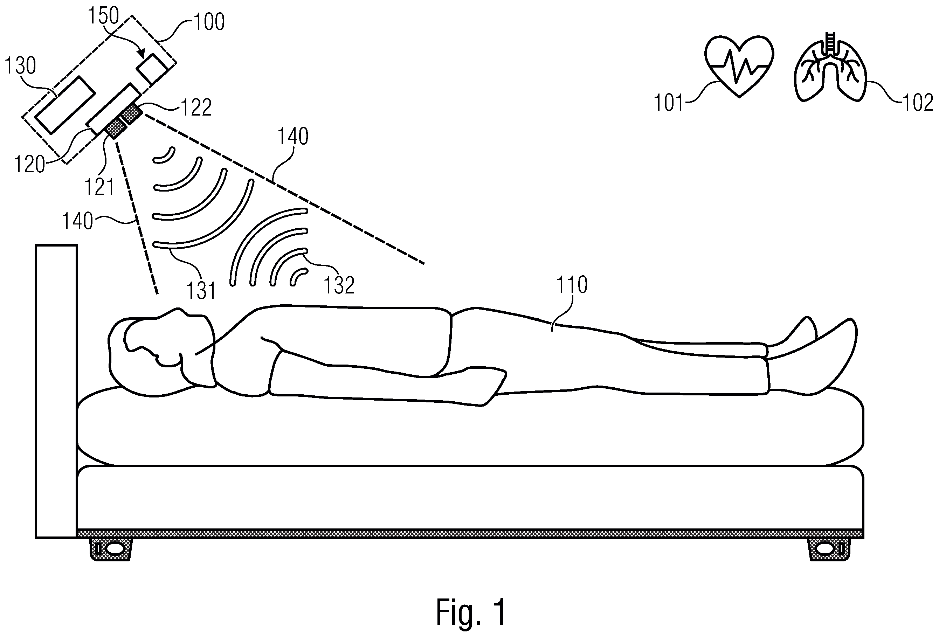

Figur 1 zeigt eine beispielhafte Ausführungsform einer erfindungsgemäßen Vorrichtung 100 zur kontaktlosen, radarbasierten Distanzmessung zu einer zu untersuchenden Objektoberfläche 110. In diesem nicht-limitierenden Ausführungsbeispiel handelt es sich bei der zu untersuchenden Objektoberfläche 110 um eine Körperregion eines menschlichen Körpers bzw. eines Körperteils. Es wäre aber auch denkbar, dass Distanzen zu anderen Objektoberflächen, wie z.B. an Maschinen, Bauteilen, Robotern und dergleichen, untersucht werden.

Hierfür weist die Vorrichtung 100 ein Radargerät 120 auf. Das Radargerät 120 weist einen Radartransmitter 121 zum Aussenden von Primär-Radarwellen 131 auf die zu untersuchende Objektoberfläche 110 auf. Das Radargerät 120 weist ferner einen Radarempfänger 122 zum Empfangen von an der zu untersuchenden Objektoberfläche 110 reflektierten Sekundär-Radarwellen 132 auf.

Die Vorrichtung 100 weist zudem eine Auswerteeinheit 130 auf, die ausgestaltet ist, um basierend auf den empfangenen Sekundär-Radarwellen 132 eine Distanz zu der Objektoberfläche 110 zu ermitteln. Die Auswerteeinheit 130 kann zum Zwecke der Datenverarbeitung beispielsweise einen Mikrocontroller, einen integrierten Schaltkreis, o.ä. aufweisen. Die Auswerteeinheit 130 kann in der Vorrichtung 100 integriert sein. Alternativ könnte die Auswerteeinheit 130 in einem externen Gerät, wie z.B. einem Smartphone, Notebook, o.ä., vorgesehen sein, wobei dieses externe Gerät mit der Vorrichtung 100 verbindbar ist.

Wie in Figur 1 zu sehen ist, ist das Radargerät 120 von der zu untersuchenden Objektoberfläche 110 beabstandet, sodass die zur Ermittlung von Bewegungen durchzuführenden radarbasierten Messungen kontaktlos erfolgen können. Vorzugsweise kann das Radargerät 120 so angeordnet sein, dass die ausgesendeten Primär-Radarwellen 131 in einem vorgegebenen Neigungswinkel, und somit schräg, z.B. im Falle eines Körpers vom Kopf in Richtung der Füße, auf den zu untersuchenden Körper treffen.

Die bei der Distanzmessung erhaltenen Daten repräsentieren einen aktuellen Abstand bzw. eine aktuelle Distanz des Radargeräts 120 zu der zu untersuchenden Objektoberfläche 110. Werden bei einer Distanzmessung voneinander abweichende Daten erhalten, so deutet dies darauf hin, dass sich im Zeitraum der durchgeführten Distanzmessung die zu untersuchende Objektoberfläche 110 relativ zum Radargerät 120 bewegt hat. Hierüber kann somit auf eine Bewegung der zu untersuchenden Objektoberfläche 110 rückgeschlossen werden.

Mit der innovativen Vorrichtung 100 können beispielsweise Bewegungen der zu untersuchenden Objektoberfläche 110 detektiert werden, die im Makrobereich liegen und somit auch mit bloßem Auge erkennbar sind, wie z.B. die Bewegung einer Gliedmaße. In dem in Figur 1 abgebildeten Ausführungsbeispiel können also zum Beispiel Bewegungen des zu untersuchenden Körpers bzw. Bewegungen eines Körperteils detektiert werden. Die detektierten Bewegungen können dann hinsichtlich einer Diagnosestellung ausgewertet werden. Beispielsweise können Beinbewegungen detektiert werden, um z.B. Bewegungsmuster des Restless Leg Syndroms zu erkennen.

Es ist aber auch möglich, Bewegungen mit deutlich kleineren Amplituden bzw. Ausschlägen zu detektieren, die mit dem bloßen Auge nicht so einfach erkennbar sind. So können beispielsweise oberflächliche Hautbewegungen detektiert werden, die sich lediglich im Millimeter- oder Mikrometerbereich abspielen. Hierüber wiederum können dann ein oder mehrere Vitalparameter des menschlichen Körpers abgeleitet werden, wie zum Beispiel der Herzschlag 101 oder die Atemfrequenz 102. Zu den detektierbaren Vitalparametern zählen im Rahmen der vorliegenden Offenbarung aber auch Parameter des vaskulären Systems, wie z.B. der Blutdruck.

In dem in Figur 1 abgebildeten Beispiel ist die zu untersuchende Objektoberfläche 110, d.h. der Körper, in der Horizontalen, also liegend, dargestellt. Es ist aber natürlich auch denkbar, dass sich der Körper in einer anderen als der hier exemplarisch abgebildeten horizontalen Position befindet. Eine erfolgreiche Distanzmessung zu der zu untersuchenden Objektoberfläche 110 hängt dabei hauptsächlich von der Orientierung bzw. Ausrichtung des Radargeräts 120 relativ zu der zu untersuchenden Objektoberfläche 110 ab. Diese Ausrichtung des Radargeräts 120 kann den Anwender jedoch gelegentlich vor Probleme stellen, da weder die ausgesendeten Primär-Radarwellen 131 noch die von der Objektoberfläche 110 reflektierten Sekundär-Radarwellen 132 sichtbar sind.

Das Radargerät 120 weist einen vordefinierten Radarmessbereich auf, innerhalb dessen das Radargerät 120 die Distanz zu der zu untersuchenden Objektoberfläche 110 ermitteln kann. Der Radarmessbereich bestimmt sich dabei u.a. durch den Ausbreitungspfad der vom Radartransmitter 121 ausgesendeten Primär-Radarwellen 131 sowie durch den Empfangs-Korridor, in dem der Radarempfänger 122 die reflektierten Sekundärwellen 132 empfangen kann.

Der in Figur 1 exemplarisch dargestellte Radarmessbereich 140 ist jedoch, ebenso wie die Radarwellen 131, 132 selbst, für den Anwender unsichtbar. Der Radarmessbereich 140 des Radargeräts 120 ist daher in Figur 1 symbolisch mittels gestrichelter Linien angedeutet.

Sofern das Radargerät 120 derart zu der zu untersuchenden Objektoberfläche 110 ausgerichtet ist, dass der Radarmessbereich 140 die Objektoberfläche 110 erfasst, können plausible Daten generiert werden, um eine Distanzmessung zu der zu untersuchenden Objektoberfläche 110 zu ermöglichen. In der hier exemplarisch abgebildeten Ausführungsform könnten beispielsweise, basierend auf einer Distanzmessung, oberflächliche Hautbewegungen an der Körperoberfläche 110 detektiert werden, um hierüber Vitalparameter 101, 102, wie z.B. den Puls oder Herzschlag, zu messen.

Nun kann es jedoch passieren, dass das Radargerät 120 nicht optimal positioniert ist, d.h. dass das Radargerät 120 ungenau gegenüber der zu untersuchenden Objektoberfläche 110 ausgerichtet ist. Der Radarmessbereich 140 deckt dann die zu untersuchende Objektoberfläche 110 nicht ausreichend ab, um genügend plausible Daten zu erfassen, die eine erfolgreiche Distanzmessung erlauben würden. Die erhaltenen Daten können in diesem Fall von der Auswerteeinheit 130 als nicht plausibel eingestuft werden, sodass die Auswerteeinheit 130 diese Daten verwirft und nicht für die Distanzmessung zu der zu untersuchenden Objektoberfläche 110 verwendet. Dies kann beispielsweise der Fall sein, wenn das Radargerät 120 in einem zu flachen Winkel auf die zu untersuchende Objektoberfläche 110 gerichtet wird.

Zusammenfassend kann also festgehalten werden, dass das Radargerät 120 nicht beliebig um die zu untersuchende Objektoberfläche 110 herum positioniert, bzw. relativ zu der zu untersuchenden Objektoberfläche 110 orientiert bzw. ausgerichtet werden kann. Um plausible Daten für eine Distanzmessung erfassen zu können, muss das Radargerät 120 vielmehr derart zu der zu untersuchenden Objektoberfläche 110 positioniert bzw. ausgerichtet werden, dass der Radarmessbereich 140 des Radargeräts 120 die zu untersuchende Objektoberfläche 110 zu einem ausreichenden Grad abdeckt.

Da jedoch, wie eingangs erwähnt, sowohl der Radarmessbereich 140 als auch die Radarwellen 131, 132 selbst, für den Anwender unsichtbar sind, kann es für den Anwender in manchen Situationen schwierig sein, das Radargerät 120 korrekt gegenüber der zu untersuchenden Objektoberfläche 110 auszurichten.

Um dieses Problem zu lösen, wird erfindungsgemäß eine optische Positionierungshilfe 150 vorgeschlagen. Diese hilft dem Anwender dabei, das Radargerät 120 korrekt gegenüber der zu untersuchenden Objektoberfläche 110 auszurichten, sodass plausible Messdaten erhalten werden können, um Distanzmessungen zu der zu untersuchenden Objektoberfläche 110 erfolgreich durchführen zu können. Hierfür ist die optische Positionierungshilfe 150 ausgestaltet, um dem Anwender den ansonsten unsichtbaren Radarmessbereich 140 des Radargeräts 120 mit Hilfe von optischen Mitteln visuell darzustellen.

Die optische Positionierungshilfe 150 dient dazu, den Anwender dabei zu unterstützen, das Radargerät 120 relativ zu der zu untersuchenden Objektoberfläche 110 derart zu positionieren, dass der visualisierte Radarmessbereich 140 auf die zu untersuchende Objektoberfläche 110 gerichtet ist, und zwar derart, dass Bewegungen Distanzmessungen zu der zu untersuchenden Objektoberfläche 110 erfolgreich durchgeführt werden können.

Um die erfindungsgemäße Positionierungshilfe 150 bestmöglich mit dem Radargerät 120 zu nutzen, ist es von Vorteil, wenn die Positionierungshilfe 150 mit dem Radargerät 120 in einer Funktionseinheit kombiniert ist. Beispielsweise können das Radargerät 120 und die optische Positionierungshilfe 150 zusammen in einem gemeinsamen Gehäuse verbaut sein, sodass bei einer Bewegung des Gehäuses sowohl das Radargerät 120 als auch die optische Positionierungshilfe 150 gemeinsam in die gleiche Richtung ausgerichtet werden. Dabei wäre es beispielsweise denkbar, das Radargerät 120 und die optische Positionierungshilfe 150 optional auf einer gemeinsamen Platine zu integrieren.

Das Radargerät 120 und die optische Positionierungshilfe 150 können somit also bei einer Bewegung des Gehäuses gemeinsam in die gleiche Richtung orientiert werden. Somit ändert sich bei einer Veränderung der Ausrichtung des Radargeräts 120 relativ zu der zu untersuchenden Objektoberfläche 110 auch die Position des visualisierten Radarmessbereichs 140 relativ zu der zu untersuchenden Objektoberfläche 110. Oder anders ausgedrückt, kann durch eine Bewegung bzw. Repositionierung des Radargeräts 120 auch der visualisierte Radarmessbereich 140 bewegt bzw. repositioniert werden.

Zur Visualisierung des Radarmessbereichs 140 des Radargeräts 120 kann die optische Positionierungshilfe 150 beispielsweise eine Lichtquelle aufweisen, die ausgestaltet ist, um einen den Radarmessbereich 140 des Radargeräts 120 repräsentierenden Lichtkegel oder Lichtstrahl auf die zu untersuchende Objektoberfläche 110 zu emittieren. Der Radarmessbereich 140 kann dabei beispielsweise direkt auf die zu untersuchende Objektoberfläche 110 projiziert werden. Dies kann beispielsweise in Form einer auf die zu untersuchende Objektoberfläche 110 projizierten Fläche realisiert werden, die dem Radarmessbereich 140 des Radargeräts 120 entspricht.

Alternativ oder zusätzlich wäre es denkbar, dass die optische Positionierungshilfe 150 ausgestaltet ist, um eine oder mehrere Messregionen, die z.B. mittels Messpunkte oder Messflächen dargestellt werden können, auf die zu untersuchende Objektoberfläche 110 zu projizieren. Diese projizierten Messregionen können dem Anwender anzeigen, wo der visualisierte Radarmessbereich 140 auf der zu untersuchenden Objektoberfläche 110 zu positionieren ist, um die Bewegungen der zu untersuchenden Objektoberfläche 110 erfolgreich detektieren zu können.

Bei der zuvor erwähnten Lichtquelle kann es sich beispielsweise um eine einen definierten Lichtstrahl erzeugende Laserlichtquelle handeln. Alternativ dazu könnte es sich bei der Lichtquelle aber auch um eine Streulichtquelle mit einem definierten Lichtkegel handeln. Ein Beispiel hierfür wäre eine handelsübliche Lampe.

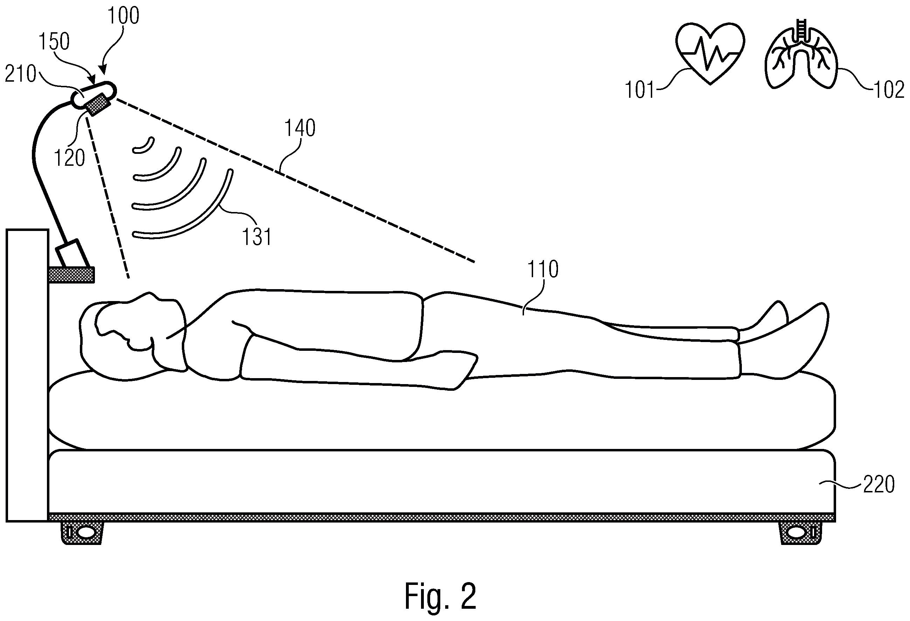

Figur 2 zeigt ein nicht-limitierendes Ausführungsbeispiel, in dem die erfindungsgemäße Vorrichtung 100 neben dem Radargerät 120 eine Lichtquelle 210 in Form einer Lampe aufweist. Eine solche Ausgestaltung ist insbesondere für den Heimanwender von Vorteil, da dies eine sehr unkomplizierte Ausrichtung der erfindungsgemäßen Vorrichtung 100 auf die zu untersuchende Objektoberfläche 110 ermöglicht. Die Lichtquelle 210 kann dabei beispielsweise in der Nähe der zu untersuchenden Objektoberfläche 110 aufgestellt werden. Wenn beispielsweise eine Körperoberfläche einer liegenden Person untersucht werden soll, so könnte die Vorrichtung 100 in der Nähe eines Betts 220 aufgestellt werden.

Wie hier zu sehen ist, kann die Lichtquelle 210 ausgestaltet sein, um den von ihr ausgehenden Lichtkegel oder Lichtstrahl (z.B. im Falle eines Lasers), der den Radarmessbereich 140 visualisiert, direkt auf die zu untersuchende Objektoberfläche 110 zu emittieren. Dies bietet eine einfache Möglichkeit, um den Radarmessbereich 140 des Radargeräts 120 direkt auf der zu untersuchenden Objektoberfläche 110 zu visualisieren bzw. anzuzeigen.

In einer denkbaren Ausgestaltung kann die Vorrichtung 100 bzw. die optische Positionierungshilfe 150 ein Signal ausgeben, falls das Radargerät 120 nicht korrekt zu der zu untersuchenden Objektoberfläche 110 ausgerichtet sein sollte, d.h. falls der visualisierte Radarmessbereich 140 so zu der zu untersuchenden Objektoberfläche 110 ausgerichtet ist, dass keine plausiblen Radardaten zum Durchführen einer Distanzmessung zu der zu untersuchenden Objektoberfläche 110 erfasst werden können.

Mittels eines unterschiedlichen zweiten Signals kann die optische Positionierungshilfe 150 anzeigen, wenn das Radargerät 120 korrekt zu der zu untersuchenden Objektoberfläche 110 ausgerichtet ist, d.h., dass der visualisierte Radarmessbereich 140 so zu der zu untersuchenden Objektoberfläche 110 ausgerichtet ist, dass plausible Radardaten zum Durchführen einer Distanzmessung zu der zu untersuchenden Objektoberfläche 110 erfasst werden können.

Das erste und zweite Signal können jeweils optische Signale sein. Dabei wäre es denkbar, dass das erste optische Signal eine erste Lichtfarbe, eine erste Lichthelligkeitsstufe, oder eine erste Blinkfrequenz ist. Das zweite optische Signal könnte dann dementsprechend eine von der ersten Lichtfarbe unterschiedliche zweite Lichtfarbe, eine von der ersten Lichthelligkeitsstufe unterschiedliche zweite Lichthelligkeitsstufe, oder eine von der ersten Blinkfrequenz unterschiedliche zweite Blinkfrequenz sein.

So wäre es beispielsweise vorstellbar, dass ein rotes Lichtsignal angezeigt wird, wenn das Radargerät 120 nicht korrekt zu der zu untersuchenden Objektoberfläche 110 ausgerichtet ist. Ein grünes Lichtsignal könnte hingegen anzeigen, dass das Radargerät 120 korrekt zu der zu untersuchenden Objektoberfläche 110 ausgerichtet ist. Andere Farben sind natürlich ebenfalls denkbar. Beispielsweise könnte das optische Signal durch Ampelfarben, oder auch durch einen Lichtübergang dargestellt werden. Es wäre dabei prinzipiell denkbar, dass das optische Signal direkt im Lichtschein des visualisierten Radarmessbereichs 140 ausgegeben wird. Dementsprechend könnte der Lichtschein selbst unterschiedliche Farben, Helligkeitsstufen oder Blinkfrequenzen aufweisen.

Alternativ oder zusätzlich zu den soeben erwähnten optischen Signalen wäre es denkbar, dass das erste Signal ein erstes akustisches Signal umfasst, und dass das zweite Signal ein von dem ersten akustischen Signal unterschiedliches zweites akustisches Signal umfasst. Beispielsweise könnte ein langsames Piepen darauf hindeuten, dass das Radargerät 120 nicht korrekt zu der zu untersuchenden Objektoberfläche 110 ausgerichtet ist. Ein schnelleres Piepen könnte hingegen darauf hindeuten, dass das Radargerät 120 korrekt zu der zu untersuchenden Objektoberfläche 110 ausgerichtet ist. Dies wäre in etwa vergleichbar mit einer Parkdistanzkontrolle in einem Fahrzeug.

In einer weiteren denkbaren Ausgestaltung kann die Vorrichtung 100 eine Rückmeldung über den visualisierten Radarmessbereich 140 erhalten. Beispielsweise kann eine Rückmeldeeinheit mit einer Kamera vorgesehen sein. Die Rückmeldeeinheit könnte beispielsweise in der Auswerteeinheit 130 integriert sein. Mit der Kamera kann der visualisierte Radarmessbereich 140 aufgenommen werden. Basierend auf den Kameraaufnahmen kann die Rückmeldeeinheit dann eine Rückmeldung zur aktuellen Positionierung des visualisierten Radarmessbereichs 140 relativ zu der zu untersuchenden Objektoberfläche 110 generieren.

Diese Rückmeldung könnte beispielsweise eine Information darüber enthalten, wo der visualisierte Radarmessbereich 140 auf der zu untersuchenden Objektoberfläche 110 aktuell positioniert ist, und/oder ob der visualisierte Radarmessbereich 140 eine Distanzmessung, z.B. zum Zwecke einer Detektion von Bewegungen der zu untersuchenden Objektoberfläche 110, ermöglicht, und/oder in welche Richtung der visualisierte Radarmessbereich 140 bewegt werden müsste, um eine Distanzmessung zu der zu untersuchenden Objektoberfläche 110 zu verbessern oder zu ermöglichen.

Gemäß einer denkbaren Ausgestaltung könnte die optische Positionierungshilfe 150 demnach ausgestaltet sein, um basierend auf der Rückmeldung zur aktuellen Positionierung des visualisierten Radarmessbereichs 140 einen visuellen Marker auf die Objektoberfläche 110 zu projizieren, der dem Anwender eine Korrekturanweisung zur Repositionierung des Radargeräts 120 einblendet. Der optische bzw. visuelle Marker kann beispielsweise eine Richtungsinformation enthalten, die dem Anwender anzeigt, in welche Richtung das Radargerät 120 bewegt werden muss, damit es korrekt gegenüber der zu untersuchenden Objektoberfläche 110 ausgerichtet ist. Hierfür kann der optische bzw. visuelle Marker beispielsweise einen Richtungspfeil enthalten.

Der optische bzw. visuelle Marker kann ein graphisches Symbol, wie z.B. ein Fadenkreuz, ein räumlich eingegrenztes Gebiet (Kreis, Viereck, etc.), einen Richtungspfeil, einen Buchstaben, eine Zahl, oder ähnliches aufweisen. Auch eine Projektion von digital angezeigten Sensorwerten - also Zahlenwerten - wäre denkbar.

Es ist aber auch möglich, dem Anwender nicht nur Korrekturanweisungen einzublenden, sondern die Korrektur der Ausrichtung des Radargeräts 120 relativ zu der zu untersuchenden Objektoberfläche 110 automatisiert durchzuführen. Hierfür könnte beispielsweise eine Regelungseinrichtung vorgesehen sein, die ausgestaltet ist, um basierend auf der Rückmeldung zur aktuellen Positionierung des visualisierten Radarmessbereichs 140 das Radargerät 120, und damit auch den visualisierten Radarmessbereich 140, bei Bedarf eigenständig zu repositionieren.

Ein weiteres denkbares Ausführungsbeispiel sieht eine dynamische Analyse der Messfähigkeit durch die Auswerteeinheit 130 vor, und damit verbunden eine Rückmeldung an das visualisierende System.

Ein konkretes Anwendungsbeispiel wäre die Erkennung einer optimalen Position bzw. eines optimalen Messorts auf der Objektoberfläche 110 für die Erfassung einer vorbestimmten Zielgröße. Diese optimale Position / Messort /räumliches Segment kann demnach auch subsummiert werden als eine für die Bestimmung einer Zielgröße optimierte Messregion.

Die optimierte Messregion kann dem Anwender somit anzeigen, wo er den Radarmessbereich 140 des Radargeräts 120 am besten positionieren sollte, um plausible bzw. bestmögliche Messergebnisse zur Ermittlung der jeweiligen Zielgröße (basierend auf der oben beschriebenen Abstandsmessung) zu erhalten.

Beispielsweise könnte die Zielgröße die Erkennung eines Restless Legs Syndroms sein, wobei in diesem Fall die optimierte Messregion im Bereich der Beine läge. Hierzu ist es hilfreich, die Lage der Beine zu kennen oder die Bewegung eines Beins zu detektieren, selbst wenn die Beine (als Beispiel einer zu untersuchenden Objektoberfläche 110) für das menschliche Auge verdeckt sind, z.B. von einer Bettdecke. Eine für die Bestimmung einer Zielgröße (z.B. Erkennung von Beinbewegungen) optimierte Messregion ist für den Anwender somit nicht immer sofort und offensichtlich erkennbar, da er z.B. nicht unter die Bettdecke schauen kann, weshalb die korrekte Positionierung des Radargeräts 120 für den Anwender oft erschwert sein kann. Das Radargerät 120 hingegen kann mit Hilfe der aufgenommenen Radardaten sozusagen durch Objekte hindurch "sehen", sodass die jeweils zu untersuchende Objektoberfläche 110 (z.B. die Beine) selbst dann erkennbar ist, wenn sie es für das menschliche Auge nicht wäre.

Ein entsprechendes Ausführungsbeispiel sieht daher vor, dass die Vorrichtung 100 ausgestaltet ist, um basierend auf von dem Radargerät 120 ermittelten Radardaten eine für die Bestimmung einer Zielgröße optimierte Messregion zu definieren. Diese optimierte Messregion kann dann auf die zu untersuchende Objektoberfläche 110 projiziert werden, um sie dort für den Anwender sichtbar zu machen. Die Projektion der optimierten Messregion auf die Objektoberfläche 110 kann den Anwender dabei unterstützen, den visualisierten Radarmessbereich 140 möglichst genau auf die angezeigte optimierte Messregion auszurichten.

Die Projektion kann beispielsweise mittels der optischen Positionierungshilfe 150 oder einer anderen Projektionsvorrichtung erfolgen.

Die auf die Objektoberfläche 110 projizierte optimierte Messregion kann beispielsweise in Form eines beliebigen geometrischen Elements, wie z.B. in Form eins Vierecks, auf die Objektoberfläche 110 projiziert werden. Dabei kann es vorstellbar sein, dass beispielsweise in bestimmten Bereichen der Objektoberfläche 110, wie z.B. im Bereich der Beine, die projizierte optimierte Messregion größer ausfallen kann als in anderen Bereichen der Objektoberfläche 110, wie z.B. im Bereich des Brustkorbs. Außerdem ist es denkbar, dass die projizierte optimierte Messregion größer ausfallen kann als die Körperregion (z.B. Beine) selbst, um z.B. Beinschlagen zu detektieren.

Dies kann somit auch für die Ausrichtung des Radargeräts 120 relevant sein, weil bei einer Bewegung der Beine diese aus dem Radarmessbereich 140 heraus bewegt werden könnten. Die Projektion der optimierten Messregion auf die Objektoberfläche 110 erlaubt somit also eine Abschätzung einer optimalen Positionierung des Radargeräts 120 unter Berücksichtigung eines "Puffers" für eventuelle Bewegungen.

Ein anderer Anwendungsfall wäre die Abbildung bzw. Projektion mehrerer optimierter Messregionen auf der Objektoberfläche 110, wobei diese mehreren Messregionen für die Erfassung einer bestimmten Zielgröße (z.B. Pulsschlag) optimiert sein können. Beispielsweise könnten die mehreren Messregionen bestimmten Körperregionen (z.B. Armen, Beinen, oder Hals) am menschlichen Körper zugeordnet sein, an dem sich die Zielgröße, wie z.B. der Pulsschlag, besonders gut ableiten lässt.

Falls beispielsweise der Pulsschlag an einer ersten Körperregion, z.B. am Hals, besser erfassbar ist als an einer unterschiedlichen zweiten Körperregion, z.B. an den Unterarmen, so kann die Auswerteeinheit 130 die erste Körperregion (z.B. Hals) als die bestmögliche auf die Zielgröße (z.B. Pulsschlag) hin optimierte Messregion festlegen und diese Messregion auf die entsprechende Körperregion projizieren.

Alternativ oder zusätzlich wäre es denkbar, dass die Auswerteeinheit 130 eine unterschiedliche zweite Körperregion (z.B. Unterarme) als die nächst-bestmögliche auf die Zielgröße (z.B. Pulsschlag) hin optimierte Messregion festlegen kann, um dann ebendiese Messregion auf die entsprechende Körperregion projizieren.

Dabei wäre es denkbar, dass lediglich eine einzige optimierte Messregion auf die Objektoberfläche (z.B. Körper) 110 projiziert wird. Dies kann die zuvor erwähnte bestmögliche optimierte Messregion (z.B. Hals) sein, oder aber auch eine beliebige andere optimierte Messregion (z.B. Unterarme, Oberarme, Beine, etc.).

Es wäre aber ebenfalls denkbar, dass mehrere optimierte Messregionen zugleich auf die Objektoberfläche (z.B. Körper) 110 projiziert werden. So könnte beispielsweise sowohl die bestmögliche Messregion (z.B. Hals) als auch die nächst-bestmögliche Messregion (z.B. Arme) sowie optional weitere best-mögliche Messregionen auf die Objektoberfläche (z.B. Körper) 110 projiziert werden.

Prinzipiell kann die Auswerteeinheit 130 eine Abstufung vornehmen, um die auf die jeweilige Zielgröße hin optimierten Messregionen hinsichtlich ihrer jeweiligen Eignung zur Bestimmung der Zielgröße zu klassifizieren, d.h. wie gut kann wo die Zielgröße detektiert werden. Die Eignung kann beispielsweise anhand der zu erwartenden Genauigkeit der Messergebnisse festgelegt werden. Wenn also zum Beispiel die besten Messergebnisse zur Bestimmung der Zielgröße (z.B. Pulsschlag) in der ersten Körperregion (z.B. Hals) erwartet werden, dann wird diese erste Körperregion als die bestmögliche optimierte Messregion festgelegt. Wenn die zweitbesten Messergebnisse zur Bestimmung der Zielgröße (z.B. Pulsschlag) in der zweiten Körperregion (z.B. Unterarme) erwartet werden, dann wird diese zweite Körperregion als die zweitbestmögliche optimierte Messregion festgelegt, und so weiter.

Wie soeben erwähnt, kann also beispielsweise eine erste Körperregion (z.B. Hals) besser geeignet sein, um eine bestimmte Zielgröße (z.B. Pulsschlag) zu ermitteln, als eine unterschiedliche zweite Körperregion (z.B. Unterarme). Diese Abstufung bzw. Klassifizierung der Eignung der jeweiligen Körperregionen, oder genauer gesagt der damit verbundenen Messregionen, kann dem Anwender ebenfalls visualisiert werden.

Zum Beispiel könnte die bestmögliche optimierte Messregion (z.B. Hals) in einer ersten Farbe (z.B. grün) dargestellt werden, eine nächstbestmögliche (z.B. zweitbestmögliche) optimierte Messregion (z.B. Unterarme) könnte in einer unterschiedlichen zweiten Farbe (z.B. orange) dargestellt werden, und eine wiederum nächstbestmögliche (z.B. drittbestmögliche) optimierte Messregion (z.B. Oberschenkel) könnte in einer wiederum unterschiedlichen dritten Farbe (z.B. rot) dargestellt werden. Neben unterschiedlichen Farben sind aber auch andere unterschiedliche Lichtcharakteristiken, wie z.B. unterschiedliche Helligkeitswerte, unterschiedliche Blinkfrequenzen, usw. denkbar.

Zwar wurden bisher Ausführungsformen beschrieben, in denen die mehreren Messregionen auf ein- und dieselbe Zielgröße hin optimiert waren. Es ist aber natürlich ebenso denkbar, dass mehrere Messregionen auf zwei oder mehr unterschiedliche Zielgrößen (z.B. Pulsschlag, Blutdruck, Beinbewegungen) hin optimiert sind und entsprechend der obigen Diskussion auf die Objektoberfläche (z.B. Körper) 110 projiziert und optional klassifiziert werden können.

Die Projektion bzw. visuelle Anzeige mehrerer, auf eine oder mehrere Zielgrößen hin optimierter, Messregionen (z.B. optimiert für die Erfassung des Pulsschlags oder des Blutdrucks) inklusive ihrer jeweiligen Eignung, basierend auf ihrer zu erwartenden Messgenauigkeit (z.B. visualisiert mittels Ampelfarben), kann die Positionierung des Radargeräts 120 relativ zur Objektoberfläche 110 unterstützen. Gerade bei der Erfassung mehrerer Zielgrößen, wie z.B. Puls, Blutdruck oder Beinbewegungen, kann die Anzeige der Messqualität je Zielgröße einen Anwender dabei unterstützen, einen optimalen Kompromiss für die best-mögliche gleichzeitige Erfassung aller Zielgrößen zu finden.

Zum Zwecke der oben beschriebenen Abstufung bzw. Klassifizierung kann die Auswerteeinheit 130 die einzelnen Messregionen hinsichtlich der zu bestimmenden Zielgröße (z.B. Pulsschlag) kategorisieren, indem sie beispielsweise mittels einer Probemessung ermittelt, wie gut die jeweilige Messregion für die Bestimmung der Zielgröße geeignet ist.

Dabei wäre es in einer ersten möglichen Ausgestaltung denkbar, dass die Auswerteeinheit 130 die Objektoberfläche 110 virtuell segmentiert, d.h. die Objektoberfläche 110 in mehrere einzelne Messregionen unterteilt, und dann in mehreren einzelnen Messregionen (vorzugsweise in jeder Messregion) jeweils eine Probemessung hinsichtlich der zu erfassenden Zielgröße (z.B. Pulsschlag) durchführt. Hierfür könnten beispielsweise in den einzelnen Messregionen Bewegungen (z.B. im Bereich der zu erwartenden Pulsfrequenz) detektiert werden. Die Messregion, in der die größte Bewegungsamplitude beobachtet wird, kann sodann als die auf die jeweilige Zielgröße hin optimierte bestmögliche Messregion klassifiziert und entsprechend auf die Objektoberfläche 110 projiziert werden. Weitere Abstufungen bzw. Klassifizierungen der einzelnen Messregionen anhand ihrer Bewegungsamplituden können dann entsprechend folgen. Die einzelnen klassifizierten Messregionen stellen somit abgestufte optimierte Messregionen dar. Sofern es sich bei der Objektoberfläche 110 um einen Körper handelt, so entsprächen diese Messregionen den oben beispielhaft beschriebenen Körperregionen bzw. Teilen davon.

In einer denkbaren Ausgestaltung kann die Vorrichtung 100 beispielsweise ein virtuelles 3D-Modell des Objekts mit der zu untersuchenden Objektoberfläche 110 erstellen. Beispielsweise kann ein 3D-Körpermodell erstellt werden. Optional kann das 3D-Köpermodell dann segmentiert, d.h. in mehrere einzelne Messregionen bzw. Körperregionen (z.B. Kopf, Brust, Beine, etc.), unterteilt werden, um die auf eine oder mehrere bestimmte Zielgrößen hin optimierten Messregionen festzulegen, wie es zuvor beschrieben wurde.

Alternativ oder zusätzlich zur Segmentierung wäre es zum Zwecke der zuvor erwähnten Abstufung bzw. Klassifizierung mehrerer Messregionen auch denkbar, dass die Vorrichtung 100 die zu untersuchende Objektoberfläche 110 analysiert bzw. abscannt, oder aber auch bereits vorab kennt. Im Falle eines Körpers könnte beispielsweise die Lage bzw. Orientierung des Körpers 110 analysiert werden. Basierend hierauf könnten dann nur an a priori bekannten sinnvollen Messregionen bzw. Körperregionen (wie z.B. im Halsbereich) Probemessungen durchgeführt werden.

In einer weiteren alternativen oder zusätzlichen Ausgestaltung wäre es denkbar, dass die Vorrichtung 100 ausgestaltet ist, um beim Erhalt von etwa gleichguten Ergebnissen innerhalb von zwei oder mehreren unterschiedlichen Messregionen eine bestimmte Messregion aufgrund einer vordefinierten Charakteristik zu favorisieren. Es könnte also beispielsweise denkbar sein, dass eine bestimmte Zielgröße (z.B. Pulsschlag) sowohl in einer ersten Messregion (z.B. Halsbereich), als auch in einer zweiten Messregion (z.B. Unterarm) und optional sogar in einer dritten oder weiteren Messregionen (z.B. Beine) in Etwa gleichgut ermittelbar ist.

Nun könnte die Vorrichtung 100 jedoch eine der Messregionen, z.B. die erste Messregion (Halsbereich), favorisieren und als die bestmögliche optimierte Messregion festlegen, da dort im Vergleich zu den anderen Messregionen bestimmte Charakteristiken auftreten oder nicht auftreten. In diesem Fall könnte diese vordefinierte Charakteristik beispielsweise eine Bewegung der jeweiligen Körperregion sein, d.h. der Halsbereich wird gegenüber den Armen und Beinen favorisiert, da sich der Halsbereich in der Regel weniger bewegt als die Arme und Beine, weshalb eine Pulsschlag-Detektion im Halsbereich besser durchführbar ist.

Nachfolgend soll der oben beschriebene Erfindungsgegenstand nochmals in anderen Worten zusammenfassend erläutert werden:
Im Wesentlichen ist das zugrundeliegende technische Problem der Erfindung die Schwierigkeit, kontaktlose, radarbasierte Sensorik intuitiv und präzise zu der zu untersuchenden Objektoberfläche 110, wie z.B. zu einem Körper, auszurichten. Da der Radarmessbereich 140 visuell nicht nachvollziehbar ist, ist die korrekte Positionierung des Radarsensors 120 erschwert. Die Erfindung nutzt Lichtschein oder allgemein optische Orientierungshilfen 150, um den Radarmessbereich 140 des Radargeräts 120 visuell anzuzeigen, was eine optimale Ausrichtung ermöglicht. Dabei ist vorgesehen, dass die Radartechnologie mit einer optischen Orientierungshilfe (z.B. Lampe, Laser) kombiniert wird. Nach der Ausrichtung kann die Lampe bzw. der Lichtschein ausgeschaltet werden, während das Radargerät 120 weiterhin Messungen durchführt.

Die vorliegende Erfindung beschreibt eine Lösung, bei der der Lichtschein, oder allgemein optische Orientierungshilfen 150, zur optimalen Positionierung des Radargeräts 120 bzw. des Radarmessbereichs 140 eingesetzt werden. Der Radarsensor 120 kann hierfür mit Licht- oder Lasertechnologie kombiniert werden, wobei der Lichtschein oder die visuellen Marker so ausgerichtet werden, dass sie den Messbereich 140 des Radars 120 visualisieren.

Dadurch wird es ermöglicht, den Radarsensor 120 flexibel in der Messumgebung zu positionieren und ein Feedback darüber zu erhalten, ob die Positionierung eine erfolgreiche Messung ermöglicht. Das beschriebene Prinzip ist in den Figuren 1 und 2 dargestellt. In Figur 2 sind beispielhaft eine Lampe 210 mit integriertem Radar 120 sowie der Radarmessbereich 140 und der Lichtschein schematisch abgebildet.

Die beschriebene Erfindung löst das Problem der schwierigen Positionierung und Sichtbarkeit des Radarmessbereichs 140 bei der Verwendung von Radartechnologie zur Distanzmessung, z.B. zum Erfassen von Bewegungen an Objektoberflächen 110, was beispielsweise zur Erfassung von Vitaldaten 101, 102 eingesetzt werden kann. Durch die Nutzung des Lichtscheins zur Darstellung des Radarmessbereichs 140 wird eine intuitive und flexible Positionierung ermöglicht, um eine effektive Erfassung der Bewegungen zu erreichen.

Zusätzlich werden in der Erfindung weitere Aspekte berücksichtigt. Eine Rückmeldung zur plausiblen Sensordatenerfassung bzw. Ausrichtung des Sensors 120 und automatische Korrektur oder Korrekturanweisung für den Nutzer können in einer Projektion dargestellt werden. Hierfür kann beispielsweise eine Kamera verwendet werden, um die Projektion aufzunehmen, sowie optional eine technische Regelung, um die Sensorposition und den Radarmessbereich 140 zu korrigieren.

Die Erfindung kann demnach beispielsweise wie folgt untergliedert werden:
1. Visualisierung (z.B. durch Ausleuchtung) des Messbereichs 140 des Radargeräts 120 ohne Feedback des Radarmesssystems und nicht steuerbar.
2. Wie in 1, jedoch mit variabler Visualisierung (z.B. durch Ausleuchtung) unter Hinzunahme von Informationen des Radarsensors 120 (z.B. in Form einer "farblichen Veränderung der Ausleuchtung bzw. des Lichtscheins").
3. Wie in 1 und/oder 2, jedoch mit Korrektur der Projektion zur Optimierung der Sensorposition und des Radarmessbereichs 140, z.B. mit einem Korrekturvorschlag als Projektion, wie der Sensor 120 ausgerichtet werden sollte (z.B. weiter nach links) um optimale bzw. plausible Sensorwerte zu erhalten.
4. Wie in 3, jedoch unter Hinzunahme von 3D-Informationen (z.B. Silhouette des Körpers) aus den Radardaten zur Schätzung des optimalen Messbereichs.

Ein Vorteil des hierin beschriebenen Erfindungsgegenstands besteht u.a. darin, die Positionierung des Radars 120 intuitiv und verbessert zu ermöglichen. Der Lichtschein bzw. die Projektion gibt dem Anwender ein direktes Feedback, ob der Sensor 120 richtig zu der zu untersuchenden Objektoberfläche 110 ausgerichtet ist.

Der Erfindungsgegenstand kann in medizinischen Einrichtungen oder für den häuslichen Bereich verwendet werden, um Sensorik für die kontaktlose Erfassung von Vitaldaten, wie z.B. Herzfrequenz und Atemfrequenz, zu positionieren. Des Weiteren ist eine Kombination mit anderen medizinischen Geräten denkbar, die diese Vitalparameter 101, 102 nutzen. Die Erfindung ist besonders nützlich in Umgebungen, in denen die Sensorpositionierung eine Herausforderung darstellt.

Die vorliegende Erfindung kann beispielsweise, und insbesondere, in Form der folgenden Ausführungsformen realisierbar sein:
Gemäß einer ersten Ausführungsform wird eine Vorrichtung (100) zur kontaktlosen, radarbasierten Distanzmessung zu einer zu untersuchenden Objektoberfläche (110) vorgeschlagen, wobei die Vorrichtung (100) unter anderem ein von der Objektoberfläche (110) beabstandetes Radargerät (120) mit einem Radartransmitter (121) zum Aussenden von Primär-Radarwellen (131) auf die Objektoberfläche (110) aufweist, und mit einem Radarempfänger (122) zum Empfangen von an der Objektoberfläche (110) reflektierten Sekundär-Radarwellen (132). Die Vorrichtung (100) weist ferner eine Auswerteeinheit (130) auf, die ausgestaltet ist, um basierend auf den empfangenen Sekundär-Radarwellen (132) die Distanzmessung zu der zu untersuchenden Objektoberfläche (110) durchzuführen, wobei das Radargerät (120) einen vordefinierten Radarmessbereich (140) aufweist. Außerdem weist due Vorrichtung (100) eine optische Positionierungshilfe (150) auf, die ausgestaltet ist, um einem Anwender den ansonsten unsichtbaren Radarmessbereich (140) mit Hilfe von optischen Mitteln (210) zu visualisieren.

Gemäß einer zweiten Ausführungsform, die mit der ersten Ausführungsform kombinierbar ist, kann die optische Positionierungshilfe (150) ausgestaltet sein, um den Anwender dabei zu unterstützen, das Radargerät (120) relativ zu der zu untersuchenden Objektoberfläche (110) derart zu positionieren, dass der Radarmessbereich (140) so auf die zu untersuchende Objektoberfläche (110) gerichtet ist, dass plausible Radardaten zur Durchführung einer erfolgreichen Distanzmessung erhalten werden.

Gemäß einer dritten Ausführungsform, die mit der ersten und/oder zweiten Ausführungsform kombinierbar ist, kann die zu untersuchende Objektoberfläche (110) eine Körperregion eines menschlichen Körpers, oder zumindest eines Körperteils desselbigen, sein.

Gemäß einer vierten Ausführungsform, die mit der dritten Ausführungsform kombinierbar ist, kann das Radargerät (120) ausgestaltet sein, um mittels der Distanzmessung Bewegungen der Körperregion zu detektieren, und die Auswerteeinheit (130) kann ausgestaltet sein, um die detektierten Bewegungen hinsichtlich einer Diagnosestellung auszuwerten.

Gemäß einer fünften Ausführungsform, die mit der dritten und/oder vierten Ausführungsform kombinierbar ist, kann das Radargerät (120) ausgestaltet sein, um mittels der Distanzmessung oberflächliche Hautbewegungen der Körperregion zu detektieren, und die Auswerteeinheit (130) kann ausgestaltet sein, um basierend auf den detektierten Hautbewegungen einen Vitalparameter und/oder einen Parameter des vaskulären Systems zu bestimmen.

Gemäß einer sechsten Ausführungsform, die mit allen vorhergehenden Ausführungsformen kombinierbar ist, kann die optische Positionierungshilfe (150) eine Lichtquelle (210) aufweisen, die ausgestaltet ist, um einen den Radarmessbereich (140) repräsentierenden Lichtkegel oder Lichtstrahl auf die zu untersuchende Objektoberfläche (110) zu emittieren.

Gemäß einer siebten Ausführungsform, die mit der sechsten Ausführungsform kombinierbar ist, kann die Lichtquelle (210) eine einen Lichtkegel erzeugende Streulichtquelle sein, oder die Lichtquelle (210) kann eine einen Lichtstrahl erzeugende Laserlichtquelle sein.

Gemäß einer sechsten Ausführungsform, die mit allen vorhergehenden Ausführungsformen kombinierbar ist, kann die optische Positionierungshilfe (150) ausgestaltet sein, um den Radarmessbereich (140) in Form einer auf die zu untersuchende Objektoberfläche (110) projizierten Fläche zu visualisieren, die den Radarmessbereich (140) des Radargeräts (120) abbildet.

Gemäß einer neunten Ausführungsform, die mit allen vorhergehenden Ausführungsformen kombinierbar ist, kann die optische Positionierungshilfe (150) ausgestaltet sein, um einen oder mehrere Messpunkte auf die zu untersuchende Objektoberfläche (110) zu projizieren, um dem Anwender anzuzeigen, wo der visualisierte Radarmessbereich (140) zu positionieren ist, um die Distanzmessung zu der zu untersuchenden Objektoberfläche (110) erfolgreich durchführen zu können.

Gemäß einer zehnten Ausführungsform, die mit allen vorhergehenden Ausführungsformen kombinierbar ist, kann die optische Positionierungshilfe (150) ausgestaltet sein, um mittels einer ersten Signalausgabe anzuzeigen, dass der visualisierte Radarmessbereich (140) so zu der zu untersuchenden Objektoberfläche (110) ausgerichtet ist, dass keine plausiblen Radardaten zum Durchführen der Distanzmessung erfasst werden können, und die optische Positionierungshilfe (150) kann zudem ausgestaltet sein, um mittels einer unterschiedlichen zweiten Signalausgabe anzuzeigen, dass der visualisierte Radarmessbereich (140) so zu der zu untersuchenden Objektoberfläche (110) ausgerichtet ist, dass plausible Radardaten zur Durchführung einer erfolgreichen Distanzmessung erfasst werden können.

Gemäß einer elften Ausführungsform, die mit der zehnten Ausführungsform kombinierbar ist, kann die erste Signalausgabe mindestens eines der folgenden optischen Signale umfassen:
- eine erste Lichtfarbe,
- eine erste Lichthelligkeitsstufe,
- eine erste Blinkfrequenz,
und die zweite Signalausgabe kann mindestens eines der folgenden optischen Signale umfassen:
- eine von der ersten Lichtfarbe unterschiedliche zweite Lichtfarbe,
- eine von der ersten Lichthelligkeitsstufe unterschiedliche zweite Lichthelligkeitsstufe,
- eine von der ersten Blinkfrequenz unterschiedliche zweite Blinkfrequenz.

Gemäß einer zwölften Ausführungsform, die mit der zehnten und/oder elften Ausführungsform kombinierbar ist, kann die erste Signalausgabe ein erstes akustisches Signal umfassen, und die zweite Signalausgabe kann ein von dem ersten akustischen Signal unterschiedliches zweites akustisches Signal umfassen.

Gemäß einer dreizehnten Ausführungsform, die mit allen vorhergehenden Ausführungsformen kombinierbar ist, kann die optische Positionierungshilfe (150) ausgestaltet sein, um basierend auf einer Rückmeldung zur aktuellen Positionierung des visualisierten Radarmessbereichs (140) relativ zu der zu untersuchenden Objektoberfläche (110) einen visuellen Marker auf die Objektoberfläche (110) zu projizieren, der dem Anwender eine Korrekturanweisung zur Repositionierung des Radargeräts (120) einblendet.

Gemäß einer vierzehnten Ausführungsform, die mit allen vorhergehenden Ausführungsformen kombinierbar ist, kann die Vorrichtung (100) ferner eine Regelungseinrichtung aufweisen, die ausgestaltet ist, um basierend auf einer Rückmeldung zur aktuellen Positionierung des visualisierten Radarmessbereichs (140) relativ zu der zu untersuchenden Objektoberfläche (110) das Radargerät (120) bei Bedarf eigenständig zu repositionieren.

Gemäß einer fünfzehnten Ausführungsform, die mit der dreizehnten und/oder vierzehnten Ausführungsform kombinierbar ist, kann die Vorrichtung (100) ferner eine Rückmeldeeinheit mit einer Kamera aufweisen, die ausgestaltet ist, um den visualisierten Radarmessbereich (140) aufzunehmen, und die Rückmeldeeinheit kann ausgestaltet sein, um basierend auf den Kameraaufnahmen die Rückmeldung zur aktuellen Positionierung des visualisierten Radarmessbereichs (140) relativ zu der zu untersuchenden Objektoberfläche (110) zu generieren.

Gemäß einer sechzehnten Ausführungsform, die mit allen vorhergehenden Ausführungsformen kombinierbar ist, kann die Auswerteeinheit (130) ausgestaltet sein, um basierend auf von dem Radargerät (120) ermittelten Radardaten eine für die Bestimmung einer bestimmten Zielgröße hin optimierte Messregion zu definieren, und diese optimierte Messregion auf die zu untersuchende Objektoberfläche (110) zu projizieren, um sie dort für den Anwender sichtbar zu machen.

Gemäß einer siebzehnten Ausführungsform, die mit mindestens einer der ersten bis fünfzehnten Ausführungsformen kombinierbar ist, kann die Auswerteeinheit (130) ausgestaltet sein, um mehrere für die Bestimmung einer bestimmten Zielgröße hin optimierte Messregionen zu definieren, und die einzelnen Messregionen hinsichtlich Ihrer Eignung zur Bestimmung der Zielgröße zu klassifizieren.

Gemäß einer achtzehnten Ausführungsform, die mit der siebzehnten Ausführungsform kombinierbar ist, kann die Auswerteeinheit (130) ausgestaltet sein, um die Klassifizierung der einzelnen Messregionen auf der Grundlage von vorab durchgeführten Probemessungen vorzunehmen, wobei bei den Probemessungen ermittelt wird, wie gut die Zielgröße in den jeweiligen Messregionen ermittelbar ist.

Gemäß einer neunzehnten Ausführungsform, die mit der achtzehnten Ausführungsform kombinierbar ist, kann die Auswerteeinheit (130) ausgestaltet sein, um im Rahmen einer Probemessung die zu untersuchende Objektoberfläche (110) in einzelne Messregionen zu segmentieren und die einzelnen Messregionen dahingehend zu untersuchen, wie gut die Zielgröße in den jeweiligen Messregionen ermittelbar ist, um basierend hierauf die einzelnen Messregionen dann zu klassifizieren.

Gemäß einer zwanzigsten Ausführungsform, die mit der achtzehnten und/oder neunzehnten Ausführungsform kombinierbar ist, kann die Vorrichtung (100) ausgestaltet sein, um basierend auf einem a priori Wissen über die zu untersuchende Objektoberfläche (110), die Probemessungen an lediglich ausgewählten Abschnitten der Objektoberfläche (110) auszuführen.

Gemäß einer einundzwanzigsten Ausführungsform, die mit mindestens einer der achtzehnten bis zwanzigsten Ausführungsformen kombinierbar ist, kann die Vorrichtung (100) ausgestaltet sein, um beim Erhalt gleichguter Messergebnisse innerhalb von zwei oder mehreren unterschiedlichen Messregionen während der Durchführung einer Probemessung, eine bestimmte Messregion aufgrund einer vordefinierten Charakteristik gegenüber den anderen Messregionen zu favorisieren, und basierend hierauf die Klassifizierung der einzelnen Messregionen vorzunehmen.

Gemäß einer zweiundzwanzigsten Ausführungsform, die mit mindestens einer der siebzehnten bis einundzwanzigsten Ausführungsformen kombinierbar ist, kann die Vorrichtung (100) ausgestaltet sein, um mindestens zwei Messregionen auf die zu untersuchende Objektoberfläche (110) zu projizieren, und die mindestens zwei Messregionen entsprechend ihrer Klassifikation zu kennzeichnen.

Gemäß einer dreiundzwanzigsten Ausführungsform, die mit der zweiundzwanzigsten Ausführungsform kombinierbar ist, kann die Kennzeichnung der mindestens zwei Messregionen mittels zweier unterschiedlicher Farben geschehen.

Gemäß einer vierundzwanzigsten Ausführungsform, die mit allen vorhergehenden Ausführungsformen kombinierbar ist, kann die Vorrichtung (100) ausgestaltet sein, um ein 3D-Modell des Objekts mit der zu untersuchenden Objektoberfläche (110) zu erstellen, und die optische Positionierungshilfe (150) kann ausgestaltet sein, um basierend auf den dem 3D-Modell zugrundeliegenden Daten mindestens eine optimierte Messregion zu definieren, innerhalb derer die Distanzmessung zu der zu untersuchenden Objektoberfläche (110) erfolgreich durchführbar ist.

Gemäß einer fünfundzwanzigsten Ausführungsform, die mit allen vorhergehenden Ausführungsformen kombinierbar ist, können das Radargerät (120) und die optische Positionierungshilfe (150) zusammen in einem gemeinsamen Gehäuse verbaut sein, um bei einer Bewegung des Gehäuses gemeinsam in die gleiche Richtung orientiert zu werden.

Die oben beschriebenen Ausführungsbeispiele stellen lediglich eine Veranschaulichung der Prinzipien des hierin beschriebenen innovativen Konzepts dar. Es versteht sich, dass Modifikationen und Variationen der hierin beschriebenen Anordnungen und Einzelheiten anderen Fachleuten einleuchten werden. Deshalb ist beabsichtigt, dass das hierin beschriebene Konzept lediglich durch den Schutzumfang der nachstehenden Patentansprüche und nicht durch die spezifischen Einzelheiten, die anhand der Beschreibung und der Erläuterung der Ausführungsbeispiele hierin präsentiert wurden, beschränkt sei.

Obwohl manche Aspekte im Zusammenhang mit einer Vorrichtung beschrieben wurden, versteht es sich, dass diese Aspekte auch eine Beschreibung des entsprechenden Verfahrens darstellen, sodass ein Block oder ein Bauelement einer Vorrichtung auch als ein entsprechender Verfahrensschritt oder als ein Merkmal eines Verfahrensschrittes zu verstehen ist. Analog dazu stellen Aspekte, die im Zusammenhang mit einem oder als ein Verfahrensschritt beschrieben wurden, auch eine Beschreibung eines entsprechenden Blocks oder Details oder Merkmals einer entsprechenden Vorrichtung dar.

### Literaturverzeichnis

[Hornig et al., 2024] Hornig, L.; Szmola, B.; Pätzold, W.; Vox, J.P.; Wolf, K.I. Evaluation of Lateral Radar Positioning for Vital Sign Monitoring: An Empirical Study. Sensors 2024, 24, 3548.

## Patentansprüche

1. Vorrichtung (100) zur kontaktlosen, radarbasierten Distanzmessung zu einer zu untersuchenden Objektoberfläche (110), mit den folgenden Merkmalen:
ein von der Objektoberfläche (110) beabstandetes Radargerät (120) mit einem Radartransmitter (121) zum Aussenden von Primär-Radarwellen (131) auf die Objektoberfläche (110), und mit einem Radarempfänger (122) zum Empfangen von an der Objektoberfläche (110) reflektierten Sekundär-Radarwellen (132),
eine Auswerteeinheit (130), die ausgestaltet ist, um basierend auf den empfangenen Sekundär-Radarwellen (132) die Distanzmessung zu der zu untersuchenden Objektoberfläche (110) durchzuführen,
wobei das Radargerät (120) einen vordefinierten Radarmessbereich (140) aufweist, und
eine optische Positionierungshilfe (150), die ausgestaltet ist, um einem Anwender den ansonsten unsichtbaren Radarmessbereich (140) mit Hilfe von optischen Mitteln (210) zu visualisieren.

2. Vorrichtung (100) nach Anspruch 1,
wobei die optische Positionierungshilfe (150) ausgestaltet ist, um den Anwender dabei zu unterstützen, das Radargerät (120) relativ zu der zu untersuchenden Objektoberfläche (110) derart zu positionieren, dass der Radarmessbereich (140) so auf die zu untersuchende Objektoberfläche (110) gerichtet ist, dass plausible Radardaten zur Durchführung einer erfolgreichen Distanzmessung erhalten werden.

3. Vorrichtung (100) nach Anspruch 1 oder 2,
wobei die zu untersuchende Objektoberfläche (110) eine Körperregion eines menschlichen Körpers, oder zumindest eines Körperteils desselbigen, ist,
wobei das Radargerät (120) ausgestaltet ist, um mittels der Distanzmessung Bewegungen der Körperregion und/oder oberflächliche Hautbewegungen der Körperregion zu detektieren, und
wobei die Auswerteeinheit (130) ausgestaltet ist,
um die detektierten Bewegungen hinsichtlich einer Diagnosestellung auszuwerten, und/oder
um basierend auf den detektierten Hautbewegungen einen Vitalparameter und/oder einen Parameter des vaskulären Systems zu bestimmen.

4. Vorrichtung (100) nach einem der vorhergehenden Ansprüche,
wobei die optische Positionierungshilfe (150) eine Lichtquelle (210) aufweist, die ausgestaltet ist, um einen den Radarmessbereich (140) repräsentierenden Lichtkegel oder Lichtstrahl auf die zu untersuchende Objektoberfläche (110) zu emittieren.

5. Vorrichtung (100) nach einem der vorhergehenden Ansprüche,
wobei die optische Positionierungshilfe (150) ausgestaltet ist,
um den Radarmessbereich (140) in Form einer auf die zu untersuchende Objektoberfläche (110) projizierten Fläche zu visualisieren, die den Radarmessbereich (140) des Radargeräts (120) abbildet, und /oder
um einen oder mehrere Messpunkte auf die zu untersuchende Objektoberfläche (110) zu projizieren, die dem Anwender anzeigen, wo der visualisierte Radarmessbereich (140) zu positionieren ist, um die Distanzmessung zu der zu untersuchenden Objektoberfläche (110) erfolgreich durchführen zu können.

6. Vorrichtung (100) nach einem der vorhergehenden Ansprüche,
wobei die optische Positionierungshilfe (150) ausgestaltet ist, um mittels einer ersten Signalausgabe anzuzeigen, dass der visualisierte Radarmessbereich (140) so zu der zu untersuchenden Objektoberfläche (110) ausgerichtet ist, dass keine plausiblen Radardaten zum Durchführen der Distanzmessung erfasst werden können, und
wobei die optische Positionierungshilfe (150) ausgestaltet ist, um mittels einer unterschiedlichen zweiten Signalausgabe anzuzeigen, dass der visualisierte Radarmessbereich (140) so zu der zu untersuchenden Objektoberfläche (110) ausgerichtet ist, dass plausible Radardaten zur Durchführung einer erfolgreichen Distanzmessung erfasst werden können,
wobei die erste Signalausgabe mindestens eines der folgenden Signale umfasst:
• eine erste Lichtfarbe,
• eine erste Lichthelligkeitsstufe,
• eine erste Blinkfrequenz,
• ein erstes akustisches Signal,
und wobei die zweite Signalausgabe mindestens eines der folgenden Signale umfasst:
• eine von der ersten Lichtfarbe unterschiedliche zweite Lichtfarbe,
• eine von der ersten Lichthelligkeitsstufe unterschiedliche zweite Lichthelligkeitsstufe,
• eine von der ersten Blinkfrequenz unterschiedliche zweite Blinkfrequenz,
• ein von dem ersten akustischen Signal unterschiedliches zweites akustisches Signal.

7. Vorrichtung (100) nach einem der vorhergehenden Ansprüche,
wobei die optische Positionierungshilfe (150) ausgestaltet ist, um basierend auf einer Rückmeldung zur aktuellen Positionierung des visualisierten Radarmessbereichs (140) relativ zu der zu untersuchenden Objektoberfläche (110) einen visuellen Marker auf die Objektoberfläche (110) zu projizieren, der dem Anwender eine Korrekturanweisung zur Repositionierung des Radargeräts (120) einblendet.

8. Vorrichtung nach einem der vorhergehenden Ansprüche,
ferner aufweisend eine Regelungseinrichtung, die ausgestaltet ist, um basierend auf einer Rückmeldung zur aktuellen Positionierung des visualisierten Radarmessbereichs (140) relativ zu der zu untersuchenden Objektoberfläche (110) das Radargerät (120) bei Bedarf eigenständig zu repositionieren.

9. Vorrichtung (100) nach einem der Ansprüche 7 oder 8,
ferner aufweisend eine Rückmeldeeinheit mit einer Kamera, die ausgestaltet ist, um den visualisierten Radarmessbereich (140) aufzunehmen, und
wobei die Rückmeldeeinheit ausgestaltet ist, um basierend auf den Kameraaufnahmen die Rückmeldung zur aktuellen Positionierung des visualisierten Radarmessbereichs (140) relativ zu der zu untersuchenden Objektoberfläche (110) zu generieren.

10. Vorrichtung (100) nach einem der vorhergehenden Ansprüche,
wobei die Auswerteeinheit (130) ausgestaltet ist, um basierend auf von dem Radargerät (120) ermittelten Radardaten,
eine für die Bestimmung einer bestimmten Zielgröße hin optimierte Messregion zu definieren, und diese optimierte Messregion auf die zu untersuchende Objektoberfläche (110) zu projizieren, um sie dort für den Anwender sichtbar zu machen.

11. Vorrichtung (100) nach einem der Ansprüche 1 bis 9,
wobei die Auswerteeinheit (130) ausgestaltet ist, um mehrere für die Bestimmung einer bestimmten Zielgröße hin optimierte Messregionen zu definieren, und die einzelnen Messregionen hinsichtlich Ihrer Eignung zur Bestimmung der Zielgröße zu klassifizieren.

12. Vorrichtung (100) nach Anspruch 11,
wobei die Auswerteeinheit (130) ausgestaltet ist, um die Klassifizierung der einzelnen Messregionen auf der Grundlage von vorab durchgeführten Probemessungen vorzunehmen, wobei bei den Probemessungen ermittelt wird, wie gut die Zielgröße in den jeweiligen Messregionen ermittelbar ist, und
wobei die Auswerteeinheit (130) ausgestaltet ist, um im Rahmen einer Probemessung die zu untersuchende Objektoberfläche (110) in einzelne Messregionen zu segmentieren und die einzelnen Messregionen dahingehend zu untersuchen, wie gut die Zielgröße in den jeweiligen Messregionen ermittelbar ist, um basierend hierauf die einzelnen Messregionen dann zu klassifizieren.

13. Vorrichtung (100) nach Anspruch 12,
wobei die Vorrichtung (100) ausgestaltet ist,
um basierend auf einem a priori Wissen über die zu untersuchende Objektoberfläche (110), die Probemessungen an lediglich ausgewählten Abschnitten der Objektoberfläche (110) auszuführen, und/oder
um beim Erhalt gleichguter Messergebnisse innerhalb von zwei oder mehreren unterschiedlichen Messregionen während der Durchführung einer Probemessung, eine bestimmte Messregion aufgrund einer vordefinierten Charakteristik gegenüber den anderen Messregionen zu favorisieren, und basierend hierauf die Klassifizierung der einzelnen Messregionen vorzunehmen.

14. Vorrichtung (100) nach einem der vorhergehenden Ansprüche,
wobei die Vorrichtung (100) ausgestaltet ist, um ein 3D-Modell des Objekts mit der zu untersuchenden Objektoberfläche (110) zu erstellen, und
wobei die optische Positionierungshilfe (150) ausgestaltet ist, um basierend auf den dem 3D-Modell zugrundeliegenden Daten mindestens eine optimierte Messregion zu definieren, innerhalb derer die Distanzmessung zu der zu untersuchenden Objektoberfläche (110) erfolgreich durchführbar ist.

15. Vorrichtung (100) nach einem der vorhergehenden Ansprüche,
wobei das Radargerät (120) und die optische Positionierungshilfe (150) zusammen in einem gemeinsamen Gehäuse verbaut sind, um bei einer Bewegung des Gehäuses gemeinsam in die gleiche Richtung orientiert zu werden.
